# EUROPEAN PATENT APPLICATION

(11) **EP 0 774 510 A1**
(43) Date of publication of application: **21.05.1997**
(21) Application number: 95921970.0
(22) Date of filing: 15.06.1995
(51) Int. Cl.: C12N 5/06, C12N 15/87, C12N 15/88, C12N 15/89, A01K 67/027

(54) **UNGULATE EG CELL**

(30) Priority: 15.06.1994 JP 157944/94
(71) Applicant: MEIJI MILK PRODUCTS COMPANY LIMITED, Tokyo 104 (JP)
(72) Inventor: HASHIMOTO, Koichiro, Odawara-shi Kanagawa 250 (JP); MITANI, Tasuku, Odawara-shi Kanagawa 250 (JP); TAKAHASHI, Nobuko, Odawara-shi Kanagawa 250 (JP); NAKAO, Haruhiko, Odawara-shi Kanagawa 250 (JP); KAWASE, Eihachiro, Odawara-shi Kanagawa 250 (JP); MATSUI, Yasuhisa, Miyagi 980 (JP)
(74) Representative: VOSSIUS & PARTNER
(86) International application number: JP9501194
(87) International publication number: WO9534636

(57) **Abstract**

A porcine EG cell strain has been established which is derived from porcine primordial germ cells, has a multipotency or totipotency like that of an embryonic stem cell, and can be subcultured on feeder cells. The strain is reactive with a monoclonal antibody 4C9 prepared by using a mouse embryonic carcinoma cell F9 strain as the antigen. As the established EG cell strain can be readily subjected to gene manipulation under culture, in particular, gene targeting, it is easy to produce a variety of swine by gene manipulation.

## Description

### Field of the Invention

The present invention relates to pluripotential or EG cells derived from ungulate primordial germ cells, chimeric embryos produced using said EG cells and chimeric animals derived from said chimeric embryos.

### Background of the Invention

After the production of mouse which had been artificially transferred and integrated with foreign genes (designated transgenic mouse) by Gordon et al. in 1980 (J. W. Gordon and F. H. Ruddle: Science, 214, 1244, 1981; J. W. Gordon et al.: Proc. Natl. Acad. Sci. USA, 77, 7380, 1980) and that of 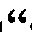 supermouse 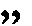 , a transgenic mouse transferred with the human growth hormone gene by Palmiter et al. in 1982 (R. D. Palmiter et al.: Nature, 300, 611, 1982), numerous attempts have been made to examine the function and expression of transferred gene at individual levels, and the production of model animals with pathosis using experimental animals such as mice and rats transferred with genes related to human genetic diseases or oncogenic genes has been contributing a great deal to the elucidation of mechanisms of genetic diseases and oncogenesis.

On the other hand, the production of transgenic ungulate animals of middle- and large-sized livestock such as cattle, swine, sheep, goat, etc. has been attempted to utilize them as so-called bioreactors which secrete physiologically active proteins usable as medicines in milk, and some of them are nearly at the stage of industrial application. For example, there have been reported a transgenic sheep called 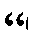 Tracy 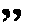 which secretes a high concentration of human α-1-antitrypsin in milk (G. Wright et al.: Bio/Technology, 9, 830, 1991; A. S. Carver et al.: Bio/Technology, 11, 1263, 1993) and a transgenic goat secreting human tissue-type plasminogen activator (K. M. Ebert et al.: Bio/Technology, 9, 835, 1991). In addition, efforts have been directed to animal breeding to enhance the milk and meat productivity essential for the livestock, make them disease-resistant and improve the feeding efficiency.

Production of such transgenic animals is usually performed by methods for directly transferring genes to nuclei of fertilized eggs. By this method, however, it is entirely impossible to arbitrarily control the exact site, copy number and orientation of integration of gene to be transferred into chromosomes. Furthermore, in general, the integration efficiency by this method is substantially low. Accordingly, it is necessary to produce whole animals from all of a large number of fertilized eggs microinjected with genes and examine these animals individually whether transferred genes are integrated and expressed, and how and where these genes are integrated in chromosomes. In addition, since middle- and large-sized livestock in general have a long pregnancy and take a long time for sexual maturation, production of transgenic domestic animals by the microinjection method inevitably requires costs and time in several years unit for breeding and maintaining a large number of them.

In addition to the microinjection method, several other methods are available for producing transgenic animals. Among them, a method for producing transgenic animals has become available utilizing ES (embryonic stem) cells, wherein said method corrects a defect of microinjection method which is unable to produce individual animals with the integration of genes into chromosomes being arbitrarily controlled.

ES cells are of cell lines obtained by culturing cells of inner cell mass (ICM), a group of undifferentiated cells, which are present in blastocyst, embryos at the developmental stage, and can differentiate into individual animals in future. ES cells were established as pluripotential cell lines in mouse in 1981 by M. J. Evans and M. H. Kaufman (M. J. Evans & M. H. Kaufman: Nature, 292, 154, 1981) followed by G. R. Martin (G. R. Martin: Proc. Natl. Acad. Sci. USA, 78, 7634, 1981). When these ES cells were transferred to normal host blastocysts followed by returning them to the uteri of pseudopregnant foster females, they yielded germ line chimeras at a high frequency (chimeric mice having functional germ cells derived from ES cells) (A. Bradley et al.: Nature, 309, 255, 1984). Various methods for transferring genes (e.g., calcium phosphate method, retroviral vector method, liposome method, electroporation method, etc.) are applicable to these ES cell lines in culture. By devising methods for selecting cells integrated with genes, it is also possible to obtain cell clones having specific targeted genes modified (by substitution, deletion or insertion) utilizing homologous recombination techniques.

Since such ES cell lines manipulated *in vitro* maintain the potential to differentiate into germ lines, active researches have been currently made to examine functions of specific genes at the individual levels (M. R. Capecchi: Science, 244, 1288, 1989). Apparently, the method for producing transgenic mice utilizing ES cells has many advantages over that by microinjection in that the former method, differing from the latter one, enables the production of individuals with arbitrary alterations only in a particular gene. Especially, it is noteworthy that it becomes possible to produce knockout animals having a specific gene inactivated, leading to the elucidation of functions of said gene and possible expression of only foreign genes. Therefore, if ES cell lines of middle- and large-sized domestic animals are established and become available for gene transfer in culture, it will enable the replacement of proteins secreted in milk with human proteins or the alteration of cell surface antigens to utilize animal's organs as donor's organs for human subjects, conceivably providing the industrial field with inestimable usefulness.

However, there are almost no reports on the successful establishment of ES cell lines in not only middle- and large-sized domestic mammals but also other animal species than mouse [M. Evans: Embryonic stem cells as a route to an experimental mammalian genetics, in "Gene Expression and its Control", Vol. 2 Genome Analysis (K. E. Daries & S. M. Tilghman, eds., pp. 1-12, Cold Spring Harbor Laboratory Press, 1991). Although reasons for this are not clear, it may be due to the fact that while ICM of blastocysts in mouse with a short gestation period proliferates rapidly, those in middle- and large-sized domestic animals grow significantly slowly and their proliferation is sometimes interrupted by the presence of resting period, resulting in a poor proliferation of ICM after planted in culture. However, the fact that the establishment of ES cell lines has not been succeeded in rat with a similar gestation period to that of mouse indicates the involvement of unknown critical factors such as growth factors.

Recently, in the attempts to culture primordial germ cells (PGCs) of mouse *in vitro,* Matsui et al. observed the formation of colonies of PGCs similar to those of ES cells in culture in the presence of LIF (leukemia inhibitory factor) and bFGF (basic fibroblast growth factor) in addition to SCF (stem cell factor, also called Steel factor, kit ligand, mast cell growth factor, etc.) as growth factors, and further succeeded in proliferating and maintaining PGCs as colonies morphologically similar to those of ES cells by using STO cells, a cell line derived from mouse embryonic fibroblasts, as feeder cells [Kawase et al. al.: Jikken Igaku (Experimental Medicine), Vol. 10, No. 13 (Suppl.), 1575-1580, 1992]. When these PGCs in culture were transferred to mouse blastocysts, they were found to be capable of forming chimeras (Matsui, Y., Zsebo, K. & Hogan, B. L. M.: Cell, 70, 841-847, 1992). It has been proposed to call such ES cell-like cells derived from PGCs as EG (embryonic germ) cells (J. L. Resmick et al.: Nature, 359, 550-551, 1992). Furthermore, it was confirmed that these EG cell lines could be established regardless of their XX or XY karyotypes and enter the germ lines (C. L. Stewart et al.: Develop. Biol., 161, 626-628, 1994). These results indicate that EG cell lines are similarly pluripotential or totipotential as ES cell lines. Since EG cell lines can be established in mice regardless of their strains, it may be possible to pave the way to obtain EG cells from the ungulate such as cattle, swine, sheep, goat, etc. by improving this method.

The present invention aims at providing EG cells derived from PGCs of the ungulate, especially swine, which are pluripotential or totipotential, and can be repeatedly subcultured *in vitro* maintaining the undifferentiated state for a long period of time. The present invention also intends to produce chimeric embryos formed using said EG cells and chimeric animals derived from said chimeric embryos. This invention further aims at forming EG cell lines having foreign genes transferred, chimeric embryos produced using said cell lines and chimeric animals derived from said chimeric embryos. In addition, the present invention aims at forming nuclear transplant embryos by transplanting nuclei of these EG cell lines into swine enucleated ooplasm and providing animals derived from said nuclear transplant embryos.

### Disclosure of the Invention

In order to attain the aforementioned objects, the present inventors completed the invention described in each item of claims. The present invention will be described in detail below using swine as an example.

Mammalian PGCs are known to have very similar migration pathways and cellular characteristics (P. D. Nieuwkoop & L. A. Sutasurya, 1979, 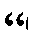 Primordial germ cells in the chordates, Embryogenesis and phylogenesis 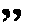 , Cambridge University Press, London). Although mice are usually used as experimental animals, their developmental mechanisms are unique among mammals in taking the mode of 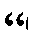 dorsoventral inverted embryo 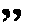 .

Most animals which take more usual developmental mode and are generally bred as livestock taxonomically belong to the ungulate based on information from comparative embryology, etc. Among ungulate domestic animals, the present inventors selected swine because they are able to become pregnant without any particular breeding season, have relatively short gestation period, are prolific to produce many fetuses, and furthermore relatively readily available.

Also it is widely known that, since the developmental mode of swine is similar to that of humans, it is often used in the medical field for such as transplantation of its skin, etc. to humans. These facts indicate that swine conceivably has more generally fundamental characteristics of mammals rather than mouse.

The term 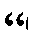 pluripotentiality 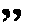 used hereinafter in the present specification means the ability of differentiating into cells derived from any germ layers comprising ectoderm, mesoderm and endoderm including germ cells, and 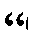 totipontiality 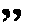 means the ability to differentiate into all cell types in future, that is, potential to form complete individual bodies.

In order to establish EG cell lines derived from swine PGCs, it is necessary to establish specific markers for identifying the existing region and number of PGCs as well as PGCs at each developmental stage, and conditions for subculturing PGCs for a long period of time.

### 1. Migration pathway and identification of PGCs

Mammalian PGCs as cells with high alkaline phosphatase (ALP) activity usually appear in the mesodermal and endodermal cell layers of the extraembryonal basal region of allantois, and migrate with the passive movement caused by the dynamic morphogenesis of embryos according to the progression of development as well as the active movement of cells themselves, subsequently reaching the genital primordium (primordial gonad, genital ridge) which will eventually develop into gonad via the epithelium of hind gut and the mesenchyme of mesentery.

Currently PGCs in several mammals including mouse have been identified using the ALP activity as the marker and their migration pathways also have been elucidated (mouse: Clark, J. M. & Eddy, E. M., Dev. biol., 47, 136-155, 1975; rat: C. H. Kemper & P. W. J. Peters, Teratology, 36, 117-124, 1987; rabbit: C. E. Chretien, Annales d'Embryologic et de Morphgenese, 1(4), 361-372, 1968; cattle: A. Jost & J. Prepin, Arch. Anat. Anat. microse., 55(2), 161-186, 1966). However, as to swine PGCs, there has been practically no information on their number and existing region (migration pathway) in relation to embryonic dpc (days post coitum) at each developmental stage, except for only one paper reporting that the genital ridge is detectable in 23-24 dpc embryos, sexuality becomes determinable from around 26 dpc, and PGCs are observed in the vicinity of mesonephros around 17 dpc (J. L. Black & B. H. Erikson, Anat. Rec., 161, 45-46, 1968).

Recently, for identifying mouse PGCs, immunohistochemical identification methods have been employed using monoclonal antibodies raised against carbohydrate chain antigens on the cell surface represented by stage-specific embryonic antigen-1 (SSEA-1), EMA-1 or Forssman antigen (Solter, D. & Knowles, B. B.: Proc. Natl. Acad. Sci. U.S.A., 45, 5565-5569, 1978; Hahnel, A. C. & Eddy, E. M.: J. Reprod. Immunol., 10, 89-110, 1987; Kanai, Y. et al.: Histochemistry, 94, 561-568, 1994). These monoclonal antibodies are produced with mouse embryonal carcinoma cells (EC cells) as antigen. Monoclonal antibody 4C9, one of those recognizing the SSEA-1-like carbohydrate chain antigen (LEX antigen) out of aforementioned antigens is produced with mouse EC cells of F9 strain as antigen (Nomoto, S. et al., Exp. Cell Res., 164, 49-62, 1980), and the immunostaining method using this antibody is given attention for its high specificity toward PGCs (Yoshinaga, K., et al.: Differentiation, 48, 75-82, 1991). In addition, anti-SSEA-1 monoclonal antibody crossreacts with mouse EG cells (Y. Matsui, et al., Cell, 70, 841-847, 1992). Based on these facts, by the immunohistochemical method using monoclonal antibody 4C9 (available under the trade name LEX-2 from Funakoshi, code Nr LE-01), the present inventors identified the existing region and number of PGCs as well as EG cell lines derived from PGCs in swine embryos, and found that said method is suitable for identifying swine EG cell lines.

### 2. Isolation of PGCs

Although there are more than 80 main breeds of swine as live stock, the present invention is not limited to these breeds. In the following, methods for isolating PGCs from swine embryos will be described.

PGCs are usually isolated from 18-39 dpc swine embryos. In general, in the case of embryos after 26 dpc, only the primordial gonad dissociated from mesonephros is used. In the case of embryos prior to this dpc, regions including a portion of mesonephros, hind gut and mesentery are used. In this case, it is preferable to remove any excessive tissues (e.g., neural tube, epidermis, liver and most portion of mesonephros) as much as possible. In case excessive tissues cannot be removed, preferable results may be obtained by once incubating dissociated cells in gelatin-coated culture dishes for 60 min and then using the cells in the culture supernatant.

Excised tissues are rinsed once with PBS(-) containing neither Ca⁺⁺ nor Mg⁺⁺, then treated with 0.25% trypsin-0.5 mM EDTA solution for 5-10 min at room temperature, and, after the addition of a serum-containing medium, the resulting mixture is repeatedly pipetted until a suspension consisting of practically single cells is obtained. Cells are recovered by centrifuging said suspension at 1,000 rpm for 5 min.

### 3. Establishment of EG cell lines

Swine EG cell lines can be established by subculturing said dissociated cells in a medium supplemented with cell growth factors on a supporting cell layer (feeder cells) comprising embryonic fibroblasts which have been inactivated by treatment with mitomycin C or γ-irradiation.

### 3·1 Feeder cells

Preparation of feeder cells are performed according to the method of Kawase et al. (Kawase et al.: Jikken Igaku, Vol. 10, No. 13 (Suppl.), 1575-1580, 1992).

As feeder cells, in addition to primary culture of swine fibroblasts, mouse embryonic fibroblasts which are feeder cells for usual mouse ES cells or the STO cell line derived from them can be used, but SL-10 cells obtained by subcloning the STO cell line are more suitable because of their better adhesiveness to gelatin. Out of STO cell line which were transferred with neomycin-resistance gene and used as feeder cells for the ES cell line, CCE, SL-10 cells were selected as those capable of most preferably maintaining said ES cells at their undifferentiated state.

In order to maintain EG cells at their undifferentiated state by subculturing them, feeder cells are preferably kept in good conditions. For this purpose, it is desirable to subculture feeder cells immediately after they reach sub-confluence (just before they reach confluence). If failed, cells tend to overlap each other and proliferate more rapidly. Cells under these conditions are generally not suitable as feeder cells. Since feeder cells tend to alter their cellular morphology and growth rate during repeated subculturing, it is advisable to abandon cells which have been in culture for about 2-3 months and newly start to culture fresh cells obtained from the frozen stock.

As culture media, those of feeder cells used for mouse ES cells can be usually used, including, for example, Dulbecco's modified Eagle's medium (DMEM) and high glucose DMEM supplemented with 10% fetal calf serum (FCS).

### 3·2 Culture method

As culture media for establishing, maintaining and proliferating swine EG cells, basically those for the mouse ES cell lines supplemented with various cell growth factors usually can be employed. For example, a basal medium consisting of high glucose DMEM supplemented with 20% fetal calf serum (FCS), 0.1 mM 2-mercaptoethanol, 30 µM nucleic acid mixture and 1,000 u/ml LIF, said basal medium which is further added with the supernatant of culture medium of Buffalo Rat liver cells (BRL-conditioned medium; BRL-CM) (Smith, A. G. & Hooper, M. L., Dev. Biol., 121: 1-9, 1987) in the ratio of 1:1 or 2:3, or said basal medium supplemented with bFGF (1-50 ng/ml), etc. can be used. In this case, the quality of water and FCS greatly influences the establishment and maintenance of EG cell lines. It is preferable to use water which is filtered through MilliQ (Milli-pore Co,) and then distilled with a quartz glass distillaton apparatus. It is also advisable to check the quality of FCS in each lot and select the one which gives good-shaped colonies of mouse ES or EG cells in a high colony-formation efficiency. In addition, it is also advisable to check the quality of FCS for effects on the growth and survival rate of mouse PGCs. Cells dissociated from a single embryo are seeded on feeder cells in one to four 35-mm culture dishes according to their dpc, and incubated under ordinary culture conditions, preferably at 37°C and under 5% CO₂ atmosphere. Small colonies are observed on feeder cells from 4 days to 1 week after the start of culture. Intercellular boundaries in these colonies are so indistinct as not easily detectable even by microscopic inspection, but nucleoli are clearly observed inside nuclei. Nuclei observed in these colonies indicate that colony forming individual cells have relatively large nuclei as compared with the size of cytoplasm, showing morphological characteristics specific to undifferentiated stem cells.

In colonies fixed with 2-4% paraformaldehyde or cold 95% ethanol, etc., intercellular boundaries become distinct clearly showing that cells closely adhere each other to form multi-layered colonies, and maintain morphological characteristics of undifferentiated stem cells.

Cells grown to sub-confluence on feeder cells are subcultured as described, for example, as follows. Cells are rinsed with PBS(-) and then trypsinized with trypsin-EDTA to form a cell suspension containing many clumps. One fourth to one second volumes of said cell suspension are seeded on feeder cell layers in fresh 35-mm culture dishes. On culturing, said clumps grow to form dome-shaped, swelling colonies. These colonies often show a strong alkaline phosphatase activity by the ordinary histochemical analysis. As these colonies gradually grow larger on continued culturing, they elongate to fine dendrites resulting in a reticular formation. In such large grown colonies are often observed regions which show only a weak or no alkaline phosphatase activity.

Also these colonies immunohistochemically react with monoclonal antibody 4C9 raised against aforementioned mouse embryonal carcinoma cell line F9 as antigen.

Swine EG cell line thus established is deposited in Kogyo Gijutsuin Seimeikogaku Kogyogijutsu Kenkyusho under Accession No. FERM BP-4694. [The cell line actually deposited is the EG cell line derived from embryos obtained from (Large White x Duroc) F1 female mated to Duroc male and at passage 4.]

### 4. Cryopreservation of swine EG cells

It is preferable to cryopreserve EG cell lines as soon as possible, because they tend to change characteristics if kept in culture for a long period of time, differentiating or decreasing the chimera forming capability.

EG cells are dissociated and preserve frozen as follows practically according to the method for subculturing said cells. After cells grown on feeder cells are first rinsed twice with PBS(-), a solution of 0.25% trypsin-0.05 mM EDTA (1 ml) is added to said cells, and they are incubated at 35-39°C for 5-10 min. After cells are dissociated by pipetting, DMEM supplemented with 10% FCS (1 ml) is added, and the cells are re-suspended by pipetting. Then, said cell suspension is transferred into a 15-ml centrifuge tube, and centrifuged at 1,000 rpm for 5 min. After removing the supernatant, pellets are suspended in a solution for cryopreservation (1 ml per 35-mm dish), and distributed 1-ml each to cryopreservation tubes (Cryotube, Nunc Co.). These cryopreservation tubes are placed as such in a -80°C deep freezer, transferred to liquid nitrogen after completely frozen, and stored (or cryopreservation tubes containing said cells are directly plunged in liquid nitrogen and preserved frozen.).

Cells kept frozen are thawed and cultured, for example, as follows. Contents of said cryopreservation tubes are thawed in a 37°C water bath, and transferred into 15-ml centrifuge tubes. After the addition of a few milliliters of DMEM supplemented with 10% FCS, the cells are re-suspended by pipetting, and then centrifuged at 1,000 rpm for 5 min. After the removal of the supernatant, pellets are suspended in a medium (2 ml per 1 cryopreservation tube), seeded onto SL10 feeder cell layers in a 35-mm dish, and cultured under similar culture conditions for ES cells.

### 5. Gene modification of swine EG cells

Since EG cells are cells in culture, various methods for transferring genes (e.g., calcium phosphate method, liposome method, microinjection method, electroporation method, etc.) can be applied to them exactly in the same manner to other cultured cells. Also there is no limitation to transferable genes including those derived from bacteria and animal or human chromosomes. Similarly, the gene modification by homologous recombination of endogenous genes using targeting vectors which is used in the gene targeting method using mouse ES cell lines is also possible. In the following is described homologous recombination method.

### 5·1 Methods for constructing targeting vectors and selecting cells with homologous recombination

Chromosomal DNAs used as the homologous region of targeting vectors preferably has the same genetic background as that of EG cells. The longer the homologous region included in vectors is, the higher the homologous recombination frequency becomes (Capecchi, M. R.: Science, 244, 1288-1292, 1989). Therefore, targeting vectors preferably contain homologous regions of more than 5 kb long. Methods for constructing vectors and selecting cells with homologous recombination comprise:
5·1·1 a method wherein vectors which contain no selection markers and are different from endogenous gene only in the base sequence serving as the primer for the PCR are used and homologous recombination events are screened from many clones obtained by the PCR (Zimmer, A. & Gruss, P.: Nature, 388, 150, 1989),
5·1·2 a method called "promotorless neo-method" wherein a promotorless vector containing neomycin-resistance gene is constructed and integrated into the exon of targeted gene with the frame being matched, and homologous recombinant cells are selected by G418-resistance (Schwartzberg, P. L. et al.: Science, 246, 799, 1989),
5·1·3 a method wherein a neomycin-resistance gene with promoter is integrated into a vector, and said vector is first transferred to cells by random and homologous recombination followed by identification of homologous recombinant cells for G418 resistance and subsequent selection of desired clone by PCR method, etc. (Joyer, A. L. et al.: Nature, 338, 153, 1989), and
5·1·4 a method called 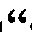 positive-negative selection method 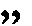 , wherein a vector is constructed with neomycin-resistance gene as the positive marker and thymidine kinase gene derived from herpesvirus (HSV-tk gene) or diphtheria toxin gene as the negative marker, and homologous recombinants are selected using G418 and Gancylovir (GANC) (Capecchi, M. R.: Science, 244, 1288, 1989; Yagi, T. et al.: Proc. Natl. Acad. Sci. U.S.A., 87, 9918-9922, 1990).

### 5·2 Transfer of targeting vectors into EG cells

Targeting vectors are usually transferred into EG cells by the microinjection method (as in the case of said method in 5·1·1) or electroporation method (as in the cases of said method in 5·1·2-5·1·4).

By methods for injecting EG cell lines with the integration of transferred genes to chromosomes being confirmed into normal embryos, chimeric swine constructed with EG cells and cells derived from normal embryos can be produced. In this case, if EG cells enter the germ line, transferred genes will be transmitted to offspring.

EG cell lines specific genes of which are modified by homologous recombination, etc. are usually heterozygous for said modified gene locus. Although EG cell lines homozygous for modified genes locus can be obtained by similar gene targeting toward said heterozygous EG cell lines, usually females and males both heterozygous for modified genes are first obtained and then homozygous offspring are produced by mating them. However, recently, there has been reported a method for obtaining directly homozygous clones in culture when ES cell clones heterozygous for modified arbitrary gene locus in mouse are selected using G418 (R. M. Mortensen et al.: Mol. Cell Biol., 12, 2391-2395, 1992). This method is also applicable to swine EG cell lines.

Since it is usually necessary to examine germ line transmission in male chimeras when the cell lines are XY, and in female chimeras when the cell lines are XX, the gender of cells is generally judged by sex chromosomal morphology. Also a method for detecting sequences specific for Y chromosome by PCR can be used. Cell lines with XY karyotype are generally used to obtain Tg mouse, because the use of male chimeras is advantageous for producing many fetuses and sperm can be cryopreserved.

### 6. Production of chimeric swine

Since EG cells which are injected into preimplantation embryos (host) are integrated in the normal development and able to differentiate into various cell types, chimeric swine can be produced by this procedure. Furthermore, if EG cell lines are germ line chimera, swine derived from EG cells can be produced.

Production of chimeric swine using EG cell lines can be performed according to the method by Kashiwazaki et al. (Veterinary Record, 130, 186-187, 1992). An example of production methods will be described below.

### 6·1 Formation and transplantation of chimeric swine embryos

Both mature and immature female swine are used for recovering host fertilized eggs. They are recovered from the uteri of female swine either artificially inseminated or spontaneously mated at their estrus induced by hormonal stimulation or in the normal estrus cycle. For the production of aggregation chimeras are used fertilized eggs at the developmental stage prior to compaction (4-8 cell division age), and for that of injection chimeras those at the stage from blastocysts to expanded blastocysts.

EG cells are trypsinized and dissociated into single cells by thorough pipetting in the medium added. Cells are seeded into culture dishes previously coated with gelatin and incubated for scores of minutes. Since feeder cells are more adhesive than EG cells, and adhere to the culture dish by this treatment, floating EG cells can be recovered.

Chimeric embryos are produced under microscope using a micromanipulator, which is equipped with a holding pipet and an injection pipet, and the zona pellucida of fertilized eggs is first held by the holding pipet. Then 10-odd EG cells are injected by the injection pipet into the perivitelline space of host fertilized eggs prior to the compaction stage in the case of aggregation chimeras, and into the blastocoel of blastocysts to expanded blastocysts in the case of injection chimeras.

Chimeric embryos are transplanted immediately after the cell injection or after several hours' incubation to the uteri of embryoreceiving swine. In this case, the estrus cycle of swine receiving transplanted embryos is adjusted to be around 1 day before or after the development of fertilized eggs. Transplantation is performed into both uterine horns or either one of them.

### 6·2 Identification of chimeric swine

When transferred chimeric embryos are conceived and delivered, live-born swine are judged to be chimeric or not. When patterns of enzymes, DNAs or fur color intrinsic to the host fertilized eggs are co-present with those intrinsic to EG cells in live-born swine, EG cells are regarded to contribute to at least phenotype expression of somatic cells. For example, if the genotype of host fertilized eggs is (Duroc x Duroc), the fur color pattern should become brown. If the genotype of EG cells is (LW x LW) (LW: large·white), the fur color pattern must become white. Therefore, if a live-born animal obtained by transferring EG cells with the genotype (LW x LW) to host fertilized eggs of the genotype (Duroc x Duroc) has partially white fur, said animal is judged to be chimeric swine.

Whether chimeric swine thus obtained are germ-line chimeras or not is proved by the inbreeding test or DNA analysis. The former test is performed as follows. Chimeric swine obtained are allowed to mature and inbred. Then genetic markers of live-born animals thus obtained are examined to determine whether said animals are produced as a result of differentiation of EG cells into germ cells of said parent chimeric swine to produce EG cell-derived sperm/ovum. For example, let us suppose that fur color pattern of a chimeric swine (e.g., female) is brown mixed with white, and that brown cells are derived from host fertilized eggs and white cells from EG cells. Said chimeric female swine is mated with a male swine of brown fur color pattern having the genotype (Duroc x Duroc). Among plural new live-born animals obtained, if there is even one swine with white fur, said chimeric swine is judged to be germ-line chimera. Furthermore, DNA analysis can be performed by isolating and analyzing sperm, ovum or germ cells from chimeric swine obtained.

### 7. Production of nuclear transplantation swine

Reproducible nuclear transplantation technique was first reported in 1983 by McGrath and Solter (McGrath, J. & Solter, D.: Science, 220, 1300-1302, 1983). With this technique, it is possible to directly obtain offspring derived from EG cells without chimera production steps. In the following, there will be described an example of the method for transplanting nuclei of EG cells in enucleated unfertilized eggs of swine.

### 7·1 Preparation of enucleated ooplasm

As the recipient cytoplasm is used enucleated ooplasm of *in vitro* or *in vivo* matured oocytes, and oocytes with pronuclei, etc.

### 7·1·1 Preparation of matured oocytes

*In vitro* or *in vivo* matured oocytes are used as matured eggs. In the former case, oocytes are isolated from follicles of excised swine ovary. Oocytes having homogeneous ooplasm and surrounded with dense cumulus cell layers are selectively collected and cultured *in vitro* to maturation. In the latter case, matured oocytes are recovered from follicles around the time of ovulation after the estrus.

### 7·1·2 Preparation of oocytes with pronuclei

When oocytes with pronuclei are used as the recipient cytoplasm, oocytes matured *in vitro* or *in vivo* are fertilized *in vitro*, or those matured and fertilized *in vivo* are recovered and used.

### 7·1·3 Preparation of enucleated ooplasm

After removing cumulus cells from oocytes matured *in vitro* or *in vivo*, oocytes having first polar bodies released are selected as matured oocytes for use. After the treatment with a medium containing inhibitors of cytoskeleton, the first polar body together with a portion of its surrounding ooplasm are removed from said matured oocytes by suction using a micropipet equipped to a micromanipulator. Finally, the completeness of enucleation is checked under a fluorescence microscope by nuclear staining using fluorescent dye such as Hoechst 33342, etc., which is DNA binding and used for vital staining. In the case where ooplasm with pronuclei are used, after pronuclei are vital stained with fluorescent dye or lipid droplets are localized by centrifugation, oocytes are enucleated using micromanipulator.

### 7·2 Preparation of donor cells

EG cells used for nuclear transplantation are prepared by dissociating them essentially according to the method for subculturing said cells. That is, cells proliferated on feeder cells are treated with trypsin solution, and then dissociated by pipetting. Then the suspension of dissociated cells mixed with feeder cells are treated in gelatin-coated dishes, and the resulting supernatant is recovered. By this treatment a considerable portion of highly adhesive feeder cells are removed, recovering EG cells in a higher yield. After said supernatant is centrifuged, pellets are rinsed and re-suspended in a small volume of medium and stored till injection.

### 7·3 Preparation of nuclear transplant embryos

For the preparation of embryos for nuclear transplantation, a method by electrofusion and another one mediated by Sendai virus (HVJ) are available. In the former case, donor cells are drawn into a micropipet equipped to the micromanipulator. Said micropipet is inserted through the incision of zone pellucida of enucleated ooplasm which are secured by a holding pipet, and donor cells are injected into the perivitelline space (injection embryo). Injection embryos are electrofused in the cell fusion medium. In the case of fusion mediated by HVJ, after the aspiration of donor cells into a micropipet followed by that of a small amount of HVJ, donor cells are injected through the micropipet gently pressed to the enucleated ooplasm so that both cells are fused by holding HVJ between them. Embryos thus treated are cultured for a while, and after the confirmation of fusion, fused embryos are further kept in culture till the time of transplantation.

### 7·4 Transplantation of nuclear transplant embryos

Nuclear transplant embryos thus prepared are cultured *in vitro*, and then the cleaved embryos are transferred to oviducts or uteri of recipients (embryos receiving swine). Estrus cycle of recipient swine is preferably adjusted to be about 1 day before or after the developing stage of nuclear transplant oocytes.

### 7·5 Examination of live-born animals derived from nuclear transplant embryos

In the case of nuclear transplant embryos, so far as the complete enucleation is confirmed by the nuclear fluorescent staining, etc. at the time of enucleation procedure, all live-born animals obtained ought to be derived from nuclear transplant embryos, except for mitochondrial DNAs etc., that is, they must exclusively have genes derived from donor cells (EG cells). Even though, due to failures in the enucleation procedure of recipient oocytes, they are not fused with donor cells by electrofusion and instead parthenogenesis is induced, it has been confirmed that these parthenogenetic oocytes cannot develop to full grown individuals, and degenerate in the midst of pregnancy. Even if these incompletely enucleated oocytes are fused with donor cells, they form triploids or tetraploids, and similarly cannot give rise to live-born animals. If at least one marker such as fur color, enzyme, DNA, etc. is available to distinguish recipient enucleated unfertilized oocytes from donor cells, it is possible to prove immediately 100% from which cells live-born animals are derived without such tedious procedures for identifying chimeras.

### 7·6 Fertility test

Live-born animals obtained by nuclear transplantation are naturally derived 100% from donor cells (EG cells) including their germ cells, and simply determined for their fertility after the maturation.

### Brief description of the drawings

Fig. 1 is a photograph showing small colonies formed about 1 week after the primary culture of swine embryonic PGCs. Intercellular boundaries within the colony are indistinct (phase contrast, object lens x40).

Fig. 2 is a photograph showing colonies after 2 week in culture (phase contrast, object lens x20).

Fig. 3 is a photograph showing colonies 2 days after passage. 3-1 was observed in bright field with object lens x10, and 3-2 in phase contrast with object lens x20.

Fig. 4 is a photograph showing colonies grown reticularly (phase contrast, object lens x10).

Fig. 5 is a photograph showing immunohistochemical staining with monoclonal antibody 4C9. Colonies of EG cells are 4C9-positive when observed by peroxidase color developing reaction (phase contrast, object lens x20).

Fig. 6 shows examples of chromosomes of swine EG cell lines 3007-5 and 3005-5.

### Best mode for carrying out the invention

Preferred embodiments of the present invention are described below, but the present invention is not limited to these embodiments.

### 1. Isolation of PGCs

### 1·1 Isolation of swine embryos

Swine embryos were obtained by mating or artificially inseminating (Large White x Duroc) F1 females with Duroc males. Some embryos were obtained by mating or artificially inseminating (Large White x Duroc) F1 females with (Duroc x France hybrid) males. In addition, embryos were obtained by mating (Duroc) swine or (Meishan) swine among themselves. Mating and artificial insemination were performed twice, once each in the morning and afternoon of the same day or in the afternoon and in the morning of next day. Days post coitum (dpc) were counted by setting the day of mating or artificial insemination at 0 dpc. Swine embryos of 18-39 dpc (18, 19, 20, 21, 22, 23, 24, 25, 26, 29, 30, 31, 32, 33 and 39 dpc) were used.

Uteri of pregnant swine were recovered immediately after slaughtering the animals, cervixes of the uteri were tied, sterilized with CETAB (cetyl trimethylammonium bromide) solution., rinsed with physiological saline, soaked in said saline in a plastic bag at room temperature (in December to April) and brought to the laboratory within 3 h. After said uteri were rinsed again with CETAB solution, embryos were taken out from uteri into physiological saline so as carefully not to damage the yolk sac and allantois of embryos as possible, and then said yolk sac and allantois were removed from said embryos.

### 1·2 Number and distribution region of PGCs in swine embryos - identification of PGCs

Using monoclonal antibody 4C9(LEX-2), immunohistochemical identification was performed with sections of swine embryos embedded in paraffin.

### 1·2·1 Method

Embryos of 20, 21.5 and 25 dpc were used.

They were treated essentially according to the method of Yoshinaga et al. (Yoshinaga, K. et al.: Differentiation, 48, 75-82, 1991) as follows. Embryos were fixed with 2% paraformaldehyde in phosphate buffer (pH 7.4) for 12 h, dehydrated with alcohol, embedded in paraffin, and then cut into 7 µm-thick serial sections. Specimens were dewaxed, soaked in methanol containing 0.3% hydrogen peroxide for 20 min to inhibit endogenous peroxidase activity. Then the sections were soaked successively in 70% and 90% alcohol for 5 min each respective ly, and further rinsed with running water for 5 min. After washing with PBS three times for 5 min each, the sections were treated with 400-fold diluted monoclonal antibody LEX-2 at 4°C for 24 h and then rinsed with PBS 3 times for 5 min each. These specimens were treated with 200-fold diluted biotin-labelled sheep anti-rat IgM antibody (Amersham) at room temperature for 45 min, rinsed with PBS three times for 5 min each, and then reacted with 100-fold diluted peroxidase-labelled streptavidin-biotin complex (Amersham) at room temperature for 30 min. After washing with PBS three times for 5 min each, specimens were soaked for 10 min in a color developing solution containing 0.02% DBA (3,3'-diaminobenzidine (Wako Pure Chemicals), 0.005% hydrogen peroxide and 50 mM sodium azide (Wako Pure Chemicals) in 50 mM Tris buffer (pH 7.5), further counterstained with hematoxylin and then microscopically inspected for LEX-2-positive cells. In control experiments, rat immunoglobulin standard serum (Bethyl) was used in place of the primary antibody.

### 1·2·2 Results

In 20 dpc embryos, LEX-2-positive cells were found mostly in the epithelium of hind gut. In addition, the positive cells were found to have already reached within the mesenchyme of dorsal mesentery and the region of presumptive gonads of mesonephros. Those LEX-2-positive cells amounted to about 70 cells. In 21.5 dpc embryos, most of the positive cells were found in the mesenchyme of dorsal mesentery and the region of presumptive gonads, amounting to about 250 cells. In 25 dpc embryos, almost all positive cells were located in the region of presumptive gonads. However, their reaction tended to become weaker, making the counting of positive cells more difficult. These results coincided with the putative distribution region of PGCs in swine embryos determined by corresponding dpc of swine with those of mouse based on their external morphology and applying the distribution region of PGCs in mouse embryos at their respective dpc to that in swine embryos. Therefore, it was proved that immunohistochemical analysis using monoclonal antibody 4C9 enables the identification of swine PGCs.

### 1·3 Isolation of swine PGCs

Said swine embryos from which yolk sac, allantois etc. were removed were transferred into PB1 solution (Wood, M. J., Whittingham, D. G. & Rail, W. F.: Mammalian Development, A Practical Approach, Monk, M. ed., pp. 255-280, IRL Press, 1987) at room temperature, and PGCs were isolated quickly by the following procedure.

In the case of embryos older than 26 dpc, only primordial gonads dissected from mesonephros were used, and in the case of embryos younger than this, the region containing a part of mesonephros, hind gut and dorsal mesentery was used. In the latter case, excessive tissues such as neural tubes, epidermis, liver and mesonephros were removed as much as possible with forceps, scissors, tungsten needle, microscalpel etc. When excessive tissues could not be removed, dissociated cells were incubated once in gelatin-coated dish for 60 min and then only cells in the supernatant were used.

Excised tissues (regions containing primordial gonads or a portion of mesonephros, hind gut and mesentery) were washed once with PBS(-), treated with 0.25% trypsin-0.5 mM EDTA for 5-10 min at room temperature, added with a serum-containing medium, and dissociated to practically single cells by pipetting. Then cells were recovered by centrifugation at 1,000 rpm for 5 min.

### 2. Establishment of EG cell lines

### 2·1 Feeder cells

The SL10 cell line, a subclone derived from the STO cell line was used. Since, in the case of SL10 cells, their usable time limit as feeder cells was only 2 weeks at most, they were passaged every 7-10 days. The medium used was DMEM supplemented with 10% FCS.

Feeder cells are prepared as follows.
2·1·1 Confluent feeder cells are inactivated by incubating in a medium (high glucose-DEME + 10% fetal calf serum) containing mitomycin C at 10 µg/ml for 2.5-3 h in a CO₂ incubator.
2·1·2 After the medium is removed, said cells are washed three times with PBS(-), detached from dishes by the treatment with 0.5% trypsin-1 mM EDTA, thoroughly suspended in the medium, and centrifuged (1,000 rpm, 5 min) to remove the supernatant.
2·1·3 Cells are re-suspended at appropriate density (in the case of STO, 2.5 x 10⁵/ml; primary culture of embryonic fibroblasts, 5.0 x 10⁵/ml).
2·1·4 Culture dishes used for the preparation of feeder cells are precoated with gelatin. They are covered with 0.1% gelatin solution (swine skin, Type A: Sigma) and incubated for more than 1 h at 37°C.
2·1·5 After the removal of said gelatin solution from dishes, said cell suspensions are transferred onto said dishes, and can be used as feeder cells after several hours.

### 2·2 Establishment and identification of EG cell lines

### 2·2·1 Cell culture

The basal medium (hereinafter designated "medium 1") used is the usual culture medium for the mouse ES cell lines shown in Table 1 (ESM) supplemented with the recombinant mouse LIF or human LIF at 1,000 U/ml. Furthermore, "medium 1" supplemented with BRL-CM in the ratio of 1/1 or 2/3 (E/B) (designated "medium 2"), and "medium 1" supplemented with bFGF at the concentration of 1-50 ng/ml (designated "medium 3") were also used. All cells recovered were cultured on feeder cells at 37°C under a 5% CO₂ in atmosphere. Culture medium were exchanged when judged to be necessary from the color of the media. Usually, the culture was initiated with the number of cells isolated from one embryo per one 35-mm culture dish.

Cells were passaged approximately according to the passage method of mouse ES cells. Cells were treated with 0.25% trypsin-0.5 mM EDTA for 3-5 min, detached from the dish by gentle pipetting in the medium added, transferred onto gelatin-coated culture dish after further pipetting, cultured for 15-30 min, and the cells in the supernatant were recovered.

**Table 1**

| Composition of ESM |
|---|
| 100 ml of DMEM |
| 0.45 g of glucose |
| 0.5 ml of NEAA (non-essential amino acid: Gibco) |
| 1 ml of nucleotide solution 1) |
| 0.1ml of 2-ME (2-mercaptoethanol) solution 2) |

| |
|---|
| 1)Nucleotide solution: aqueous solution containing 3 mM each of adenosine, guanosine, cytidine and uridine and 1 mM thymidine is heated to 37°C to make a complete solution, and sterilized by filtration, and stored at -20°C. |
| 2)2-ME solution: To PBS(-) (1 ml) is added 2-ME (7 µl) is added, and sterilized by filtration. This solution can be stored at 4°C for 1 week. |
| 3)Antibiotics are usually not used. If necessary, penicillin or streptomycin is used. |

### 2·2·2 Proliferative Morphology

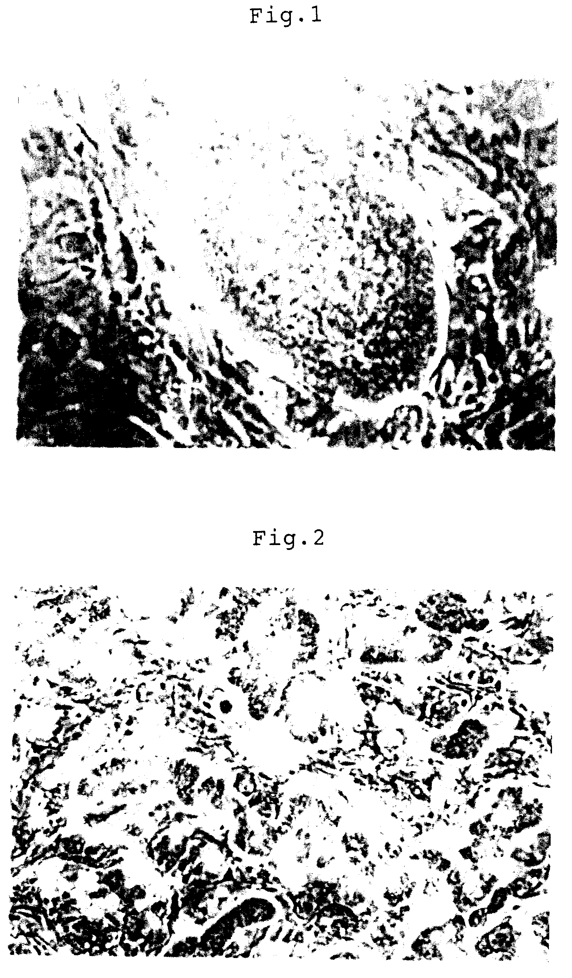

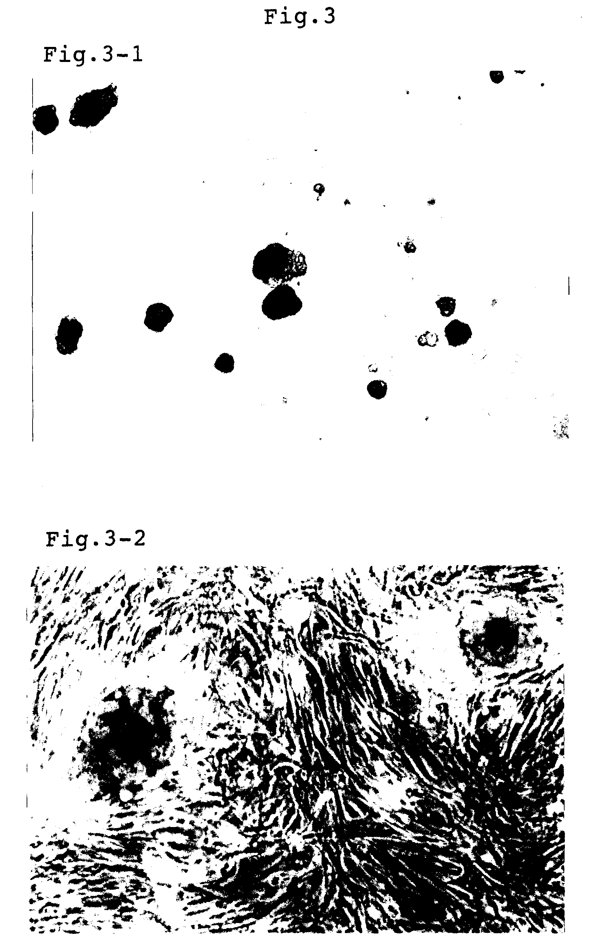

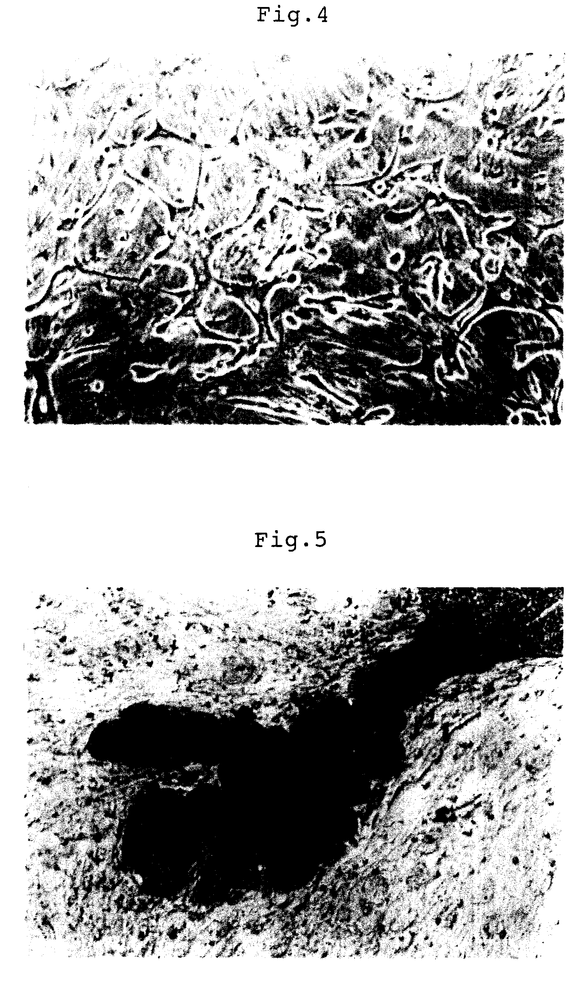

From cells of about 18-24 dpc embryos in culture, by 4 days to 1 week after the initiation of culture, swelling colonies with indistinct intercellular borderlines were observed to appear on feeder cells and elongated slenderly (Figs. 1 and 2). They were passaged after 1-2 weeks. These colonies were difficult to dissociate into single cells by usual treatment, forming many clumps, which grew to dome-shaped swelling colonies on feeder cells (Figs. 3-1, 3-2). On continued culturing, these rounded colonies elongated slenderly, and were connected each other, eventually forming reticular colonies (Fig. 4). On fixation, intercellular boundaries became distinct.

In comparison of the cells grown in said culture media 1-3, the cell growth in "medium 2" was slower than in other media, and colonies were more swelling in shape than those in "medium 1" or "medium 3". "Medium 2" and "medium 3" caused changes in the morphology of feeder cells, and especially in "medium 3" the life of feeder cells became slightly shorter. There was no significant difference in effectiveness between mouse and human LIFs. No difference was observed with LIF concentration increased up to 10,000 U/ml.

There were no differences observed among colonies of PGCs from embryos obtained by mating (Large White x Duroc) F1 female to Duroc male, among (Duroc)s and among (Meishan)s.

On continuation of passages for 6 months, a few colonies showed a tendency to differentiate, but most colonies maintained characteristics of undifferentiated cells with aforementioned morphology.

### 2·2·3 Histochemical staining of alkaline phosphatase (ALP)

After culture dishes of swine EG cell lines were washed twice with Dulbecco's PBS(-), cells were fixed with 95% cold ethanol at 4°C for more than 30 min, and then dehydrated with 4°C anhydrous ethanol for more than 30 min. After the fixing solution was removed, the dishes were rinsed three times with 0.1 M Tris-HCl (pH 9.0-9.5) at room temperature for 5 min each. Then the staining solution (see Method for preparing the staining solution below) (1.5-2 ml) was added to each dish, and ALP was stained in the dark at room temperature for 15-30 min. After rinsing twice with PBS(-), glycerol was added to dishes, colonies of EG cells stained reddish brown were inspected with phase contrast stereomicroscope. Results are shown in the ALP column of Table 2.

**Table 2**

| Properties of swine EG cell line | | | | | | |
|---|---|---|---|---|---|---|
| Cell line | Strain | dpc | Karyotype | Normality (%) | ALP | 4C9 |
| 3003-1 | LWD² | 23 | XX | 92-96 | + | + |
| 3003-6 | LWD² | 23 | XX | 76-93 | + | + |
| 3004-7 | LWD² | 23 | XY | 85-92 | ++ | ++ |
| 3005-5 | LWD² | 23 | XX | 80-100 | +++ | +++ |
| 3005-13 | LWD² | 23 | XX | 62-93 | +++ | +++ |
| 3006-4 | LWD² | 23 | XX | 71-96 | +++ | +++ |
| 3006-15 | LWD² | 23 | XX | 81-96 | +++ | +++ |
| 3006-16 | LWD² | 23 | XX | 81-93 | ++ | ++ |
| 3007-5 | LWD² | 23 | XY | 76-89 | +++ | +++ |
| 3008-12 | LWD² | 23 | XY | 85-93 | +++ | +++ |
| 3020-7 | LWD² | 23 | XY | 76-94 | ++ | ++ |
| 3022-1 | LWD² | 23 | ND | Tetraploid | ++ | ++ |
| 3042-16 | Duroc | 21 | XX | 84-96 | +++ | +++ |
| 0008 | LWD² | 21 | XX | 96 | ++ | ++ |
| 0019 | LWD² | 23 | ND | Tetraploid | ++ | ++ |
| 0093 | Duroc | 23 | XX | 86-89 | ++ | ++ |
| 0095 | Duroc | 23 | XY | 53-84 | + | + |
| 0100 | LWD² | 23 | XX | 82-92 | ++ | ++ |
| Each EG cell line which had been thawed from cryopreserv ed samples were after 1-5 passages. Degrees of ALP staining and 4C9 immunohistochemical staining are shown as follows: + weak, ++ moderate, +++ distinct, ND not determined. | | | | | | |

### •Method for preparing the staining solution

To 0.1 M Tris-HCl buffer (25 ml) were added Naphthol AS-B1 phosphate (Sigma, catalog Nr. N-2125) (2.5mg), Fast red TR salt (Sigma) (15 mg) (or Fast purple B salt (Sigma) (6 mg) and Fast blue BB salt (Sigma) (12.5 mg), and the mixture was dissolved by stirring for 3-5 min in the dark, and then filtered.

### 2·2·4 Immunohistochemical identification of cells in culture

### a. Reaction with anti-4C9 antibody

EG cell lines grown in 35-mm culture dishes were fixed with 2% phosphate buffered paraformaldehyde (pH 7.4) for 12 h at 4°C and washed with PBS. In order to inhibit the endogenous peroxidase activity, cells were soaked in methanol containing 0.3% hydrogen peroxide at room temperature for 20 min, then in 70% and 90% alcohol respectively for 5 min each, and further washed with running water for 5 min. After washing three times with PBS for 5 min each, cells were reacted with 50-100fold diluted monoclonal antibody LEX-2 at 4°C for 24 h. After washing three times with PBS at room temperature, cells were reacted with a 100-fold diluted biotin-labelled sheep anti-rat Ig antibody (Amersham) at room temperature for 45 min under rotating. After washing three times with PBS for 5 min each, cells were reacted with a 100-fold diluted peroxidase labelled streptavidin-biotin complex for 30 min at room temperature under rotating. After washing three times with PBS for 5 min each, cells were stained by soaking in the color developing solution comprising 0.02% DAB (3,3'-diaminobenzidine, Wako Pure Chemicals), 0.05% hydrogen peroxide and 50 mM sodium azide (Wako Pure Chemicals) in 50 mM Tris buffer (pH 7.5) at room temperature for 10 min. Specimens were washed with tap water and then inspected under microscope. In control experiments, rat immunoglobulin standard serum (Benthyl) was used in place of the primary antibody 4C9. Results are shown in Fig. 5. Color was developed with treated swine EG cells, confirming the binding of swine EG cells with monoclonal antibody LEX-2.

### b. Fluorescent antibody staining of anti-4C9 antibody

Examination of anti-4C9 antibody-positive specimens using fluorescent antibody was all performed by double histochemical staining for both anti-4C9 antibody and ALP. After culture dishes of swine EG cell lines were washed three times with cold PBS containing 2% fetal bovine serum (FBS) and 0.1% NaN₃, EG cell lines were fixed with 0.1 M phosphate buffer containing 2% paraformaldehyde at room temperature for 20-30 min. After washing three times with PBS, it was replaced with a blocking solution [PBS containing 5% normal goat serum (TAGO Co., code Nr. 5000)], and dishes were kept at room temperature for 20-30 min. Then, the blocking solution was replaced with M199 Hanks' solution containing 5% BSA, 250-fold diluted goat anti-rat IgM antibody (µ chain) and 0.1% NaN₃, and the dishes were kept at room temperature for 20-30 min. After washing three times with PBS, cells were reacted with 10-fold diluted LEX-2 monoclonal antibody (Funakoshi, code Nr LE-01) (40-80 µl) for about 14-26 h. After washing three times with PBS, treated cells were reacted with 25-fold diluted FITC-conjugated goat anti-rat IgM antibody [ORGANON TEKNIKA Co., code Nr 55759 (1613-0201)] (40-50 µl) for 30 min. After washing with PBS, cells were stained for alkaline phosphatase at room temperature for 5-18 min in a similar manner to that for staining mouse ES cells. After washing twice with PBS followed by DDW, dishes were whole mounted using Vectashield mounting solution (for fluorescent examination, Vector Laboratories Co., code Nr. H-1000), and cells were frame-marked using manicure solution and inspected under microscope. Results are shown in the 4C9 column of Table 2.

### 2·2·5 Karyotype analysis

Cell lines thus established are usually subjected to karyotype analysis to determine the ratio of cells having normal chromosome number. The greater the ratio of cells with normal chromosome number is, the higher the possibility of said cells to differentiate into germ cells with chimera forming capability becomes. In addition, changes in chromosome number after passages are usually examined.

### a. Cells to be examined

In order to obtain better results, swine EG cells in the logarithmic growth phase 3-7 days after passage were used. Growth medium was exchanged afresh 3-5 h prior to passage.

### b. Specimen preparation

### •Colcemid treatment

Cells in the logarithmic growth phase in 35-mm culture plates were incubated with a culture medium containing colcemid at a final concentration of 0.04 mg/ml at 37°C for 2 h in a 5% CO₂ incubator (Concentration of colcemid and its treatment time are appropriately adjusted according to the type of cells and their growth rate).

### •Hypotonic treatment

After the completion of incubation, both culture supernatant and cells which had been processed with 0.05% trypsin and recovered from each cell sample were transferred to the same centrifuge tube, and centrifuged (800-1,000 rpm, 5 min). Following the removal of the supernatant, to the cell pellets thus obtained was added a hypotonic solution (0.56% KCl solution, 3 ml) to disperse cells, and the suspension was kept at 37°C for 13 min.

### •Fixation

After the hypotonic treatment, a fixation solution (methanol:glacial acetic acid = 3:1, 9 ml) was added to the cell suspension, and the mixture was kept on ice for 20 min or at 4°C overnight. To the cells recovered by centrifugation was added the fixation solution (4 ml) and mixed. After the same procedure was repeated twice, cell pellets were suspended in the fixation fluid (100-300 µl). This cell suspension (1-2 drops) which was applied to glass slides by Pasteur pipet was kept in a slide box at room temperature overnight.

### •Staining

Cells fixed on slides were stained with freshly prepared 5% Giemsa solution in 50 mM phosphate buffer (pH 6.8) for 5 min, washed with DW for 1-2 min, and kept at room temperature.

### c. Chromosome analysis

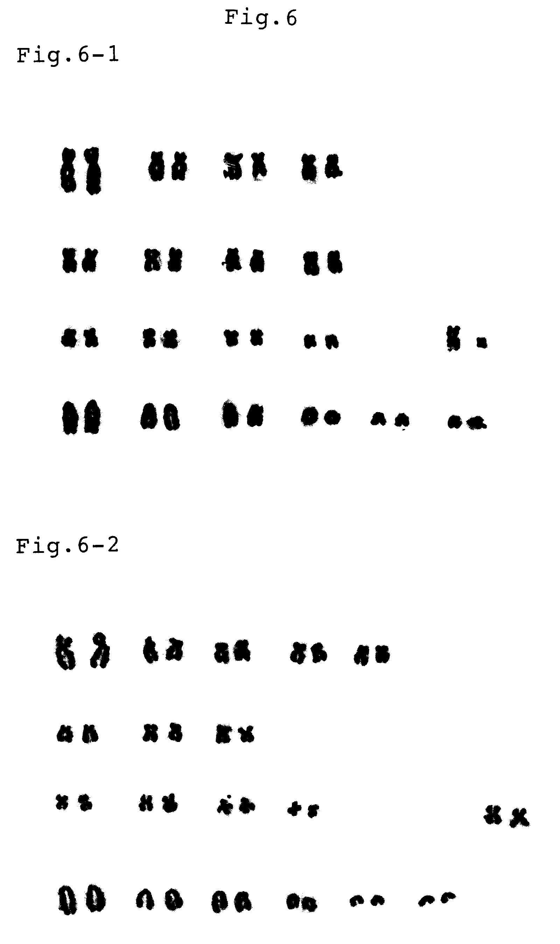

Specimens of thoroughly dispersed chromosomes of each cell line at metaphase of cell division were photographed on minicopy films (Fuji, ASA 25-32). Results are shown in the karyotype column of Table 2, and morphological characteristics of chromosomes of two cell lines are presented in Fig. 6. Fig. 6-1 is the photograph of chromosomes of swine EG cell line 3007-5 after 5 passages, and Fig. 6-2 is that after 2 passages.

### 2·2·6 Gender determination

Gender determination of swine EG cell line was performed according to the method reported by Fajfar et al. [Fajfar, C. J., A. L. Rayburn III, L. B. Schook and M. B. Wheeler (1993) Animal Biotech., 4, 183-193] with a slight modification. This modified method enables the gender determination even though cells are contaminated with feeder cells.

Genomic DNAs of swine EG cells were prepared using the standard method for extracting genomic DNAs [Sambrook, J., E. F. Fritsch and T. Maniatis (1989) Molecular Cloning A Laboratory Manual 2nd ed., CSH Press]. Amount of DNA was measured and a solution of DNA (10 ng/µl) was prepared.

MilliQ water (37.5 µl), PCR buffer (5 µl), dNTP mixed solution (4 µl), a set of primers, YH-1: 5'-GCCCTATCTTACCATGGTCAGCCC-3' (1 µl) and YH-2: 5'-CCTCACCAGCAACCTCACAGACC-3' (1 µl) and Taq polymerase (0.5 µl) in 49 µl in total were mixed in 0.5-ml Eppendorf tubes.

To the above mixture gently mixed by pipetting was added the aforementioned swine EG genomic DNAs (10 ng/µl) (1 µl) just prior to the reaction, and the solution was mixed for a short period of time. To the resulting mixture was added mineral oil (more than 60 µl). Thirty cycles of PCR were performed, wherein each cycle involved steps for 1 min at 94°C, 1 min at 60°C and 2 min at 72°C.

Gel electrophoretic analysis of PCR products showed the formation of a band having 192 bp. It was confirmed that feeder cell's DNA did not have this band, while swine sperm DNA has a distinct band of the same length.

EG cell lines 3005, 3006 and 3008 were subjected to the gender determination. PCR amplification product of DNA from 3007 and 3008 was of the same length as that of bore sperm DNA, while said product of the same length was not obtained from DNAs of 3005 and 3006, similarly not from DNA of SL10. Sincenonspecif ic amplification almost does not occur with these samples, it was concluded that karyotype of 3007 and 3008 are XY, while that of 3005 and 3006 are XX.

### 2·2·7 Induction of differentiation

To swine EG cells in 35-mm dish was added trypsin-EDTA (1 ml), and they were incubated for 3-10 min. Then, culture medium (0.2 ml) was added to them, and cell clumps were dissociated into single cells by pipetting. After the culture medium was added to dissociated cells to make a final volume of 10 ml, cells were counted, and centrifuged at 800 rpm for 5 min. Cells were re-suspended in a culture medium containing 0.1-0.3 M retinoic acid (all trans, type XX, Sigma R-2625) to a final density of 5 x 10⁵ cells/ml, and 5-ml aliquot was seeded on tissue culture dish (Falcon #3002) precoated with 0.1% gelatin solution.

Two days later, the medium was removed from the dish by aspiration, and replaced with a fresh medium containing retino ic acid.

As cells start to differentiate, their growth rate is gradually reduced. When cells are allowed to grow continuous ly, they are treated with trypsin-EDTA prior to reaching confluence, and seeded on dishes which have been coated with a gelatin solution containing the fresh medium with retinoic acid at 3 x 10⁵ cells/ml.

### Industrial applicability

Since swine EG cell lines of the present invention morphologically resemble ES cells, have pluripotentiality or totipotentiality of differentiation and are capable of being passaged for a long time with these characteristics being maintained, they can be subjected to gene manipulation in culture in a similar manner as other cultured cells, for example, gene targeting, etc. Therefore, if EG cell lines which can differentiate into germ cells are obtained, it is possible to produce chimeras by transferring industrially useful genes into said EG cell lines and combining said modified EG cells with normal embryos, and obtain EG cell line derived transgenic offsprings from said chimeras. Also EG cell derived offsprings can be obtained by transplanting nuclei of said EG cells to enucleated ooplasm using nuclear transplantation techniques.

## Claims

1. Ungulate EG cells which are derived from ungulate primordial germ cells , having the pluripotentiality or totipotentiality similar to that of embryonic stem cells and capable of being passaged on feeder cells.

2. EG cells according to claim 1 capable of reacting with a monoclonal antibody 4C9 raised against mouse embryonal carcinoma cells used as antigen.

3. EG cells according to claim 1 or claim 2 having the following characteristics:
a) said cells form small colonies 4 days to 1 week after the start of cell culture on feeder cells, having indistinct intercellular boundaries and distinct nucleoli inside their nuclei. On fixation, said intercellular boundaries in said colonies become distant, showing that said cells tightly adhere each other to form multilayered colonies, still maintaining morphological characteristics of undifferentiated stem cells;
b) on passage, said colonies are not readily dissociatable forming clumps, which form dome-shaped swelled colonies when cultured on feeder cells, and on further continued culturing said colonies elongate slenderly like dendrites as they grow eventually to reticular structures; and
c) said cells proliferate maintaining morphological characteristics of undifferentiated cells even after repeated passages over a long period of time.

4. EG cells according to claim 1 wherein said cells are derived from swine which is an ungulate.

5. Chimeric embryos produced by transferring EG cells according to claims 1, 2, 3 or 4 to normal host embryos.

6. Chimeric ungulate animals and their offsprings derived from said chimeric embryos according to claim 5.

7. EG cells produced by transferring foreign genes to cells according to any one of claims 1, 2, 3 or 4.

8. EG cells according to claim 7 wherein endogenous genes of said cells are modified with transferred foreign genes.

9. EG cells according to claim 8 wherein endogenous genes of said cells are inactivated with transferred foreign genes.

10. Chimeric embryos produced by transferring cells according to claims 7, 8 or 9 to normal host embryos.

11. Chimeras derived from said chimeric embryos according to claim 10.

12. Nuclear transplant embryos produced by transplanting nuclei of said cells according to any one of claims 1, 2, 3, 4 or claims 7, 8, 9 into enucleated unfertilized oocytes of ungulates.

13. Whole ungulate animals derived from said nuclear transplant embryos according to claim 12.
